# EUROPEAN PATENT SPECIFICATION

(11) **EP 3 998 343 B1**
(45) Date of publication and mention of the grant of the patent: **20.03.2024**
(21) Application number: 20947818.9
(22) Date of filing: 28.12.2020
(51) Int. Cl.: C12Q 1/68, C12N 15/10, C12N 15/11

(54) **DOUBLE-ENDED LIBRARY LABEL COMPOSITION AND APPLICATION THEREOF IN MGI SEQUENCING PLATFORM**
DOPPELENDIGE BIBLIOTHEKSMARKIERUNGSZUSAMMENSETZUNG UND IHRE VERWENDUNG BEI EINER MGI-SEQUENZIERUNGSPLATTFORM
COMPOSITION DE MARQUEURS DE BANQUE À DOUBLE EXTRÉMITÉ ET SON APPLICATION DANS UNE PLATEFORME DE SÉQUENÇAGE MGI.

(30) Priority: 19.08.2020 CN 202010838955
(43) Date of publication of application: 18.05.2022
(73) Proprietor: Nanodigmbio (Nanjing) Biotechnology Co., Ltd, Jiangsu 210000 (CN)
(72) Inventor: WANG, Biao, Nanjing, Jiangsu 210000 (CN); HU, Yugang, Nanjing, Jiangsu 210000 (CN); WU, Qiang, Nanjing, Jiangsu 210000 (CN)
(74) Representative: Barker Brettell LLP
(86) International application number: PCT/CN2020/139919
(87) International publication number: WO 2022/036977

(56) References cited:
- WO-A1-2020/118596
- CN-A- 109 971 827
- CN-A- 110 628 882
- CN-A- 111 534 518
- LAURAE MACCONAILL ET AL: "Unique, dual-indexed sequencing adapters with UMIs effectively eliminate index cross-talk and significantly improve sensitivity of massively parallel sequencing", BMC GENOMICS, BIOMED CENTRAL LTD, LONDON, UK, vol. 19, no. 1, 8 January 2018 (2018-01-08), pages 1-10, XP021252292, DOI: 10.1186/S12864-017-4428-5
- PANU SOMERVUO ET AL: "BARCOSEL: a tool for selecting an optimal barcode set for high-throughput sequencing", BMC BIOINFORMATICS, BIOMED CENTRAL LTD, LONDON, UK, vol. 19, no. 1, 5 July 2018 (2018-07-05), pages 1-6, XP021258232, DOI: 10.1186/S12859-018-2262-7

## Description

### Technical Field

The invention relates to the field of plasma DNA library construction, more specifically, refers to a dual library tags composition and application thereof in the MGI sequencing platform.

### Background

In the sequencing process of the MGI high-throughput sequencer, in order to realize more samples sequencing, each sample needs to be labeled with a different index and sequenced and then the data is split through bioinformatic analysis depending on the indexes information. However, at present single-end library tags are basically used in MGI sequencing platform. As single-end library tags (index) have natural defects, it is easy to cause data crosstalk problems between different samples. Due to the contamination of adapters or primers in synthesis, experimental process and sequencing, crosstalk problems are inevitable. Therefore, it is necessary to solve the low-frequency mutual crosstalk problems between different samples. The best way is to use dual library tags, which can effectively remove the mutual crosstalk problems between different samples.

However, compared with single-end library tags, applying dual library tags, whether the sequencer can accurately read the dual library tags or not, will seriously affect the effective splitting of the sequencing data through bioinformatic analysis. If there is a problem with reading the sequences of the dual library tags, the sequencing data splitting rate will be reduced, thereby increasing the sequencing cost.

Therefore, how to use dual tags to label pooled libraries, which can not only reduce the sample crosstalk problems but also improve the sequencing data splitting rate, is a problem to be solved.

### Summary

The main purpose of the invention is to provide a dual library tags composition and application thereof in MGI sequencing platform, to solve the sample crosstalk problems when using the single-end library tags in MGI sequencing platform.

In order to achieve the purpose, according to an aspect of the invention, the invention provides a dual library tags composition, and the dual library tags composition includes a plurality of 5' end library tags and a plurality of 3' end library tags, the lengths of the 5' end library tags are all the same, the lengths of the 3' end library tags are all the same, and the occurrences of each base at the same position are also all the same.

Further, the lengths of the 5' end library tags are all the same with the lengths of the 3' end library tags, preferably, are any fixed lengths between 6~10bp; preferably, in the dual library tags composition, there are at least 3 base differences between any two library tags, and the number of continuous same bases in any library tag does not exceed 3, GC contents in all library tags are all 40-60%. preferably, the dual library tags composition comprises a combination of 4-balanced dual library tags, or a combination of 8-balanced dual library tags, wherein the combination of 4-balanced dual library tags comprises 4n 5' end library tags and 4n 3' end library tags, and the combination of 8-balanced dual library tags comprises 8n 5' end library tags and 8n 3' end library tags, wherein n is an integer greater than or equal to 1.

Further, in the combination of 4-balanced dual library tags, the 5' end library tags are selected from any one or more of the 96 groups shown in Table 1, and the 3' end library tags are selected from any one or more of the 96 groups shown in Table 1 that are different from the 5'-end library tags.

Further, in the combination of 8-balanced dual library tags, the 5' end library tags are selected from any one or more of the 48 groups shown in Table 2, and the 3' end library tags are selected from any one or more of the 48 groups shown in Table 2 that are different from the 5'-end library tags.

According to the second aspect of the invention, the invention provides composition of amplification primers with dual library tags based on MGI sequencing platform, and the composition of amplification primers includes a plurality of amplification primer pairs with dual library tags, each amplification primer pair comprises a 5' end library tag and a 3' end library tag, and the lengths of multiple 5' end library tags of the amplification primer pairs are all the same, and the lengths of multiple 3' end library tags of the amplification primer pairs are all the same, and the occurrences of each base at the same position are also all the same.

Further, the lengths of multiple 5' end library tags of the amplification primer pairs are all the same with the lengths of multiple 3' end library tags of the amplification primer pairs; preferably, the lengths of the multiple 5' end library tags and the lengths of the multiple 3' end library tags are any fixed lengths between 6 ~10bp; preferably, in the composition, there are at least 3 base differences between any two library tags, and the number of continuous same bases in any library tag does not exceed 3; preferably, GC contents in all library tags are all 40-60%; preferably, the composition comprises a combination of 4n 4-balanced amplification primer pairs, or a combination of 8n 8-balanced amplification primer pairs, wherein n is an integer greater than or equal to 1.

Further, in the combination of 4n 4-balanced amplification primer pairs, the 5' end library tags are selected from any one or more of the 96 groups shown in Table 1, and the 3' end library tags are selected from any one or more of the 96 groups shown in Table 1 that are different from the 5'-end library tags; preferably, in the combination of 8n 8-balanced amplification primer pairs, the 5' end library tags are selected from any one or more of the 48 groups shown in Table 2, and the 3' end library tags are selected from any one or more of the 48 groups shown in Table 2 that are different from the 5'-end library tags.

Further, each amplification primer pair further comprises a 5' end universal amplification sequence and a 3' end universal amplification sequence, the 5' end universal amplification sequence comprises an universal upstream sequence of the 5' end library tag and an universal downstream sequence of the 5' end library tag, and the 3' end universal amplification sequence comprises an universal upstream sequence of the 3' end library tag and an universal downstream sequence of the 3' end library tag; preferably, the universal upstream sequence of the 5' end library tag is SEQ ID NO: 793, and the universal downstream sequence of the 5' end library tags is SEQ ID NO: 794; the universal upstream sequence of the 3' end library tag is SEQ ID NO: 795, and the universal downstream sequence of the 3' end library tag is SEQ ID NO: 796; or
the universal upstream sequence of the 5' end library tag is SEQ ID NO: 793, and the universal downstream sequence of the 5' end library tag is SEQ ID NO: 797; the universal upstream sequence of the 3' end library tag is SEQ ID NO: 795, and the universal downstream sequence of the 3' end library tag is SEQ ID NO: 798.

According to the third aspect of the invention, the invention also provides a sequencing library construction kit, which includes any one of the above composition of amplification primers.

Further, the kit further comprises bubble adapters, wherein the bubble adapters comprise a first adapter sequence and a second adapter sequence, the first adapter sequence is SEQ ID NO: 769, and the second adapter sequence is SEQ ID NO: 770, or the first adapter sequence is SEQ ID NO: 773, and the second adapter sequence is SEQ ID NO: 774.

According to the fourth aspect of the invention, the invention provides a method for constructing a sequencing library based on MGI sequencing platform, comprising applying any one of the kit to construct.

According to the fifth aspect of the invention, the invention provides a sequencing library including the above dual library tags combination, or any one of the above combinations of amplification primers.

By introducing the dual library tags and the optimized dual library tags combination, when applying the dual library tags for sequencing data splitting, the crosstalk problems caused by synthesis, experimental process and machine sequencing can be solved, and the results will be more accurate. Further, by controlling that the lengths of 5' end library tags and the lengths of the 3' end library tags are the same, and limiting the occurrences of each base at the same position are the same, the bases of the dual tags in the composition have the same occurrence, so when the adapters or library amplification primers with the dual tags of the composition are synthesized, multiple libraries with good base-balanced dual tags can be obtained. When these multiple libraries are pooled and sequenced on the machine, the sequences of the dual tags can be read accurately and the sequencing data can be split effectively.

### Brief Description of the Drawings

The accompanying drawings, which form a part of this application, are provided to further understand the present invention, the illustrative embodiments of the present invention and the description thereof are intended to explain the present invention and are not intended to limit thereto. In the drawings:
Figure 1A, Figure 1B, and Figure 1C show the advantages of MGI sequencing platform using dual tags over single-end tags to remove crosstalk problems;
Figures 2A and 2B show two forms of MGI single-end tag adapter;
Figures 3A and 3B show two forms of MGI dual tag adapter;
Figure 4 shows the process of constructing a library using two dual tags based on MGI platform;
Figure 5 shows that the inventions applying the dual tags of the present invention are compatible with the inventions applying the single-end tags;
Figure 6 shows an adapter in which the dual tags amplification primers and the single-end tags amplification primers are compatible;
Figures 7A and 7B show the base-balanced type of 4-balanced and 8-balanced sequences;
Figure 8 shows the comparison of base-balance between 4-balanced and 8-balanced tags in the hybrid process;
Figure 9 shows the output comparison of the two library construction methods;
Figure 10 shows the difference in sequencing data split between 4-balanced and 8-balanced tags in 12 pooled samples sequencing processes.

### Detailed Description of the Embodiments

It should be noted that the embodiments in the present application and the features in the embodiments may be combined with each other without conflict. The present invention will be described in detail below with reference to the embodiments.

### Interpretation of specific terms:

Dual tag adapters: For high-throughput sequencing, a universal sequencing adapter is required to connect to the ends of each fragment. Each non-complementary region of the adapter has a variable sequence that is a tag sequence, which is used to split data during sequencing.

Base balance of tag sequences: DNA sequence consists of four bases, namely A, T, G and C. For effective reading during sequencing, a set of tag sequences is combined to ensure the base ratio of each position in the tag sequence is equal.

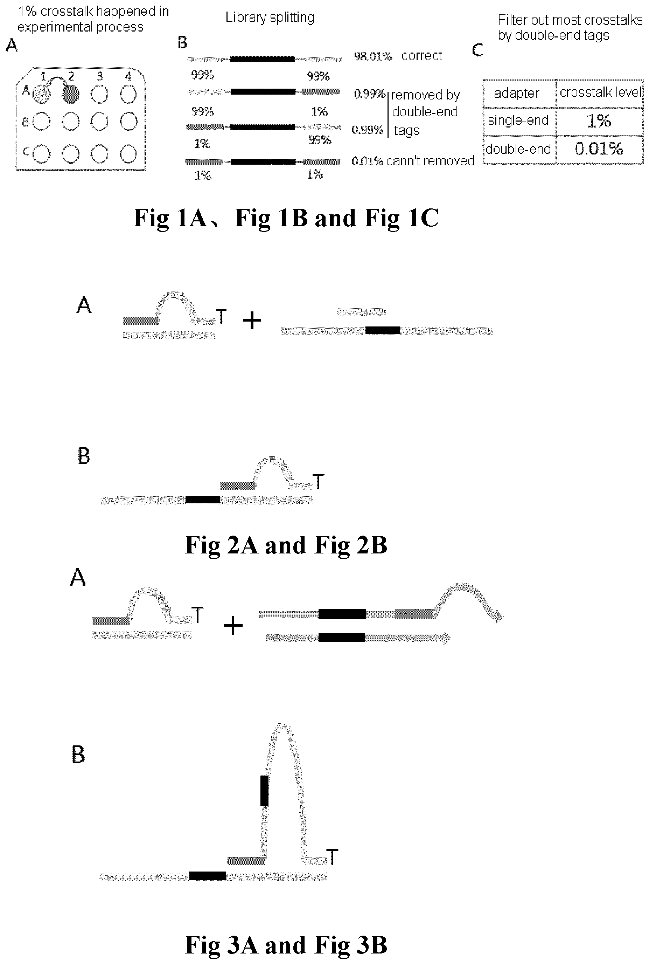

As mentioned in the background, when single-end tags are used to construct libraries for MGI high-throughput sequencing, there are some crosstalk problems between samples (this is a phenomenon that also exists in Illumina sequencing platform. Although MGI platform is much different from the Illumina platform, the process of adapter sequence synthesis, library construction, and hybridization capture inevitably causes crosstalk problems between samples). As shown in Figure 1A, if there are 1% mutual crosstalk problems in the experimental process, whether it is in adapter synthesis, library construction, hybridization capture, or machine sequencing, there will be the same crosstalk problems. The best way to solve the crosstalk problems between samples is to introduce the dual tags in the process of library construction. As shown in Figure 1B, the crosstalk problems can only be solved by introducing the dual tags meanwhile controlling experimental processes as much as possible. As shown in Figure 1C, the dual tags will reduce crosstalk problems by 100 times (1% to 0.01 %) than the single-end tag.

In order to solve the sample crosstalk problems in MGI sequencing platform, this invention also tries to change the single-end tags to the dual tags. The research and development ideas and process are as follows.

Bubble adapters are used in MGI library construction. Unlike Illumina Y-type adapters, MGI single-end tags can be fused into the adapters (as shown in Figure 2B) or separately used (Figure 2A); while the dual tag sequences cannot be fused with the front end sequence (as shown in Figure 3B, if the tag sequence is fused at the front end, since the front end region is only 7 bp, the bubble structure will be longer, and the stability of this structure is extremely poor, and the efficiency is very low. And the implementation effect is not as efficient as the truncated structure where the tag sequence primers and the universal adapter are separated). And the universal adapter and the dual tag amplification primers can be separately used (as shown in Figure 3A). The dual tags were connected according to the structure shown in Figure 3A, and inventors found the large bubble in the middle of the bubble adapter would affect the stability of the annealing secondary structure, and affect the ligation of the adapters (average efficiency is 20%-40%). MGI bubble adapter is different from Illumina Y-adapter in which the dual tags can be fused together.

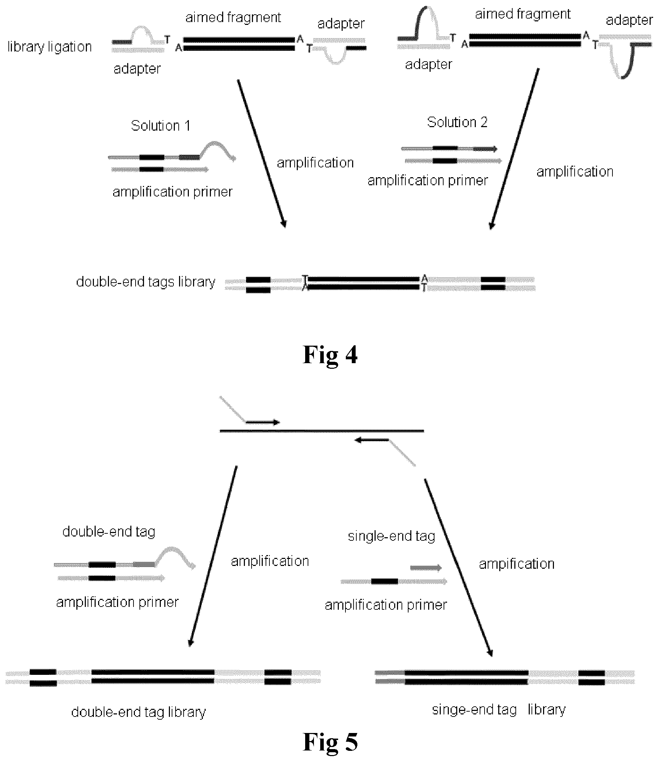

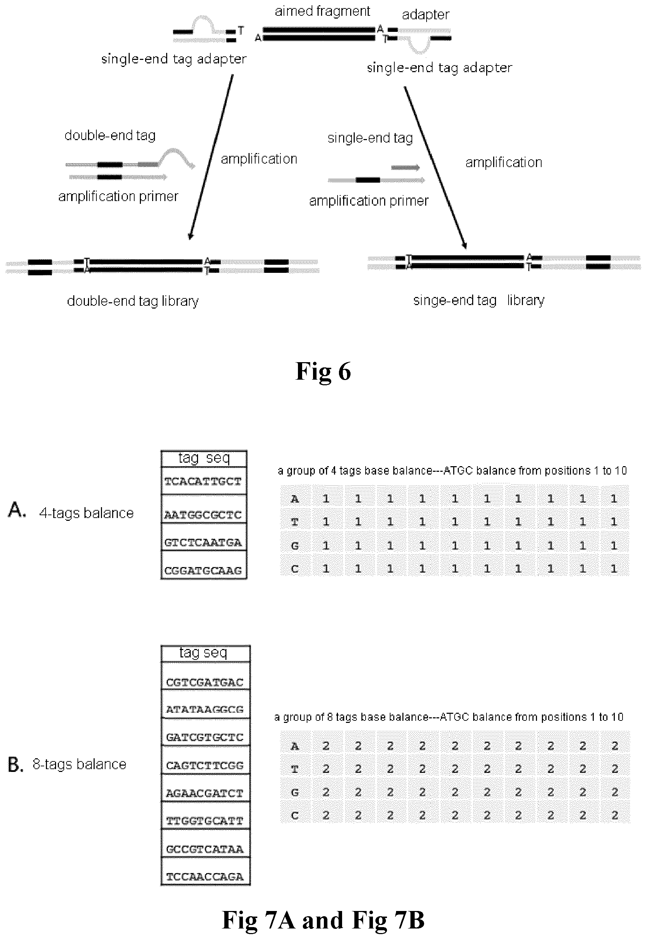

Further research found that when the unpaired bases in the middle region of the MGI bubble adapters can be 30±5bp, and the paired base is 20±2bp, it is easier to form a stable annealing ligation, improving the ligation efficiency, as shown in the Solution 1 of Figure 4. When the unpaired bases in the middle region can also be 45±5bp, and the paired base is 25±2bp, it is easier to form a stable annealing ligation, improving the ligation efficiency, as shown in the Solution 2 of Figure 4. The inventorss further found that compared with the Solution 2, the Solution 1 has the following advantages: first, when the bubble region is 30±5bp, adapters anneal stably, the region to be complementary with is short and the stability is benefit for ligation. Second, being compatible with amplicons with single-end tags, and the amplicons can be switched between single-end tags and dual tags, as shown in Figure 5. It is compatible with single-end tag adapters, as shown in Figure 6.

The inventors further found that although Solution 2 has many advantages over the Solution 1, both two solutions can work if you want to obtain the sequencing library in MGI sequencing platform with dual tags. If the constructed library with dual tags is used for machine sequencing and the sequencing data is split after sequencing, the inventors found that the base balance requirements of MGI dual tag adapters during sequencing are more stringent than that of the single-end tag adapters, and the sequencing data can only be split when the tag sequences at two ends are both correct, as shown in Figure 1B. That is, although the dual tags solve the crosstalk problems between samples, the base balance requirements for machine sequencing are extremely stringent, and the poor base balance will seriously affect the accurate reading of the sequencing data, which in turn affects the effective sequencing split.

In order to split the sequencing data more accurately, taking the base number of the dual tags are both 10 as an example, the inventors have optimized the base balance of the dual tags according to the following rules, and the rules for base screening are as follows: 1) There are 3 base differences between each tag sequence; 2) The GC content of each sequence is 0.4-0.6; 3) The number of continuous same bases cannot exceed 3. According to these rules, the secondary structure of each selected tag sequence was evaluated to see whether a secondary structure such as hairpin folds is formed between the tag sequence and the universal primer at the 3' end of the amplification primer, which will reduce the amplification efficiency, affects the balance of each tag base in the pooled sample libraries, further affects the reading accuracy of tag sequence, and therefore reduces the accuracy of sequencing data splitting.

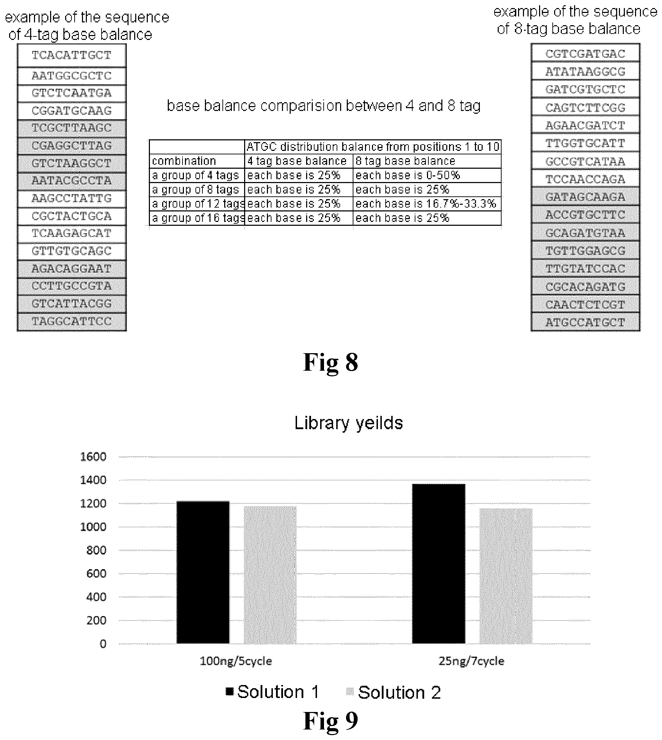

According to the above optimized screening rules, the present invention optimizes 384 types of 4-balanced tags and 384 types of 8-balanced tags sequences. 4-balanced tags refer to a group of 4 tags sequences, as shown in Figure 7A (first 1-4 tags shown in Table 4). A group of 4 tags sequences refers to base A, T, G, or C occurs once in each position from the 1^{st} to the 10^{th} position of each tag. Similarly, the 8-balanced tags refer to a group of 8 tags sequences, as shown in Figure 7B (first 1-8 tags shown in Table 5). A group of 8 tags sequences refers to base of A, T, G, or C occurs twice in each position from the 1^{st} to the 10^{th} position of each tag. According to multiple tests of the invention, the group of 4-balanced tags is the smallest unit of balance and the best combination. 4-balanced tags combinations can be combined into 4, 8, 12, and 16 combinations that are 4 fold-balanced, and 8-balanced tags combinations need to be combined into 8 and 16 combinations that are 8 fold-balanced. As shown in Figure 8 (the tag sequence of the 4-balanced tags combination on the left corresponds to the library tag combination carried by the first 4 sets of amplification primer sets in Table 1, and the library tag of the 8-balanced tags combination on the right corresponds to the library tag combination carried by the first two sets of amplification primer sets in Table 2), when the 4-balanced tags libraries are pooled and sequenced on the machine, the bases are balanced, and the proportion of each base is 25%. And when the 8-balanced tags combinations are used, the proportion of each base is 0-50%. When the 8 folds, for example, 8 or 16 samples are pooled on the machine, the proportion of each base after the library tags combination can be balanced, and each is 25%. When 12 samples are pooled and sequenced on the machine, the proportion of each base in the 8-balanced tags combination is between 16.7% and 33.3%.

In addition, the balance of non-integer fold of 4 tags is also better than the combination of 8-balanced tags, and the application of 4-balanced tags is more conducive. As the sequencing throughput of MGI sequencer becomes higher and higher, the optimized 384 types of 4-balanced tags combinations in the present application make the four close libraries with 4-balanced tags be sequenced effectively (see Table 1 for the 4-balanced tags combinations). The optimized 384 8-balanced tags combinations also make the eight close libraries with 8-balanced tags be sequenced effectively (see Table 2 for the 8-balanced tags combination).

Preferably, when the two balanced tags are used for forming the dual amplification primers, the sequence of primer 1 is a forward arrangement of 384 numbers, and the primer 2 is a reverse arrangement of 384 numbers, which is a recommended arrangement of the present invention. In practical applications, it can also be combined and arranged according to actual needs. For example, as shown in Table 1, when primer 1 is selected in any of the 96 groups, primer 2 can be selected in any of the remaining 95 groups. Of course, if the number of samples to be pooled is greater than 4, such as 8 or 12, the number of the tag groups of the primer 1 just need to be different from that of the primer 2. For example, the primer 1 are selected from the first 3 groups, and primer 2 can be selected any 3 groups from the remaining 93 groups. As long as 4 fold samples are pooled and sequenced on the machine, the dual library tags can be selected according to this rule.

When the number of the pooled samples is not integer fold of 4, the 4 samples with large amount of sequencing data shall be arranged in one set of balanced tag combinations, and the samples with small amount of sequencing data shall be arranged in another set of other balanced tag combinations. The 4-balanced tags combinations have obvious advantages over the 8-balanced tags combinations in this situation. 4-balanced tags combinations have an advantage over 8 balanced combinations for integer fold of 4 (4, 12, 20), and the combination of non-integer fold of 4 is also better than the 8-balanced tags combination, and the balance is better than that of the 8-balanced tags combination when the number of samples to be pooled is 4n+1 and 4n+2. Therefore, the 4-balanced tags combination has the following advantages: 1) The combinations of 4-balanced tags are twice as many as the 8-balanced ones; 2) For the three groups of unbalanced arrangements, the balance in the combinations of 4n+1 and 4n+2 groups is also better than the combination of 8-balanced tags; 3) When there is a difference in the amount of sequencing data between samples, the combinations of 4-balanced tags is better arranged close to the balance, and the samples for large amount of sequencing data are prioritized in the balanced combination, and it can be unbalanced for the samples for small amount of sequencing data.

**Table 1: 4-balanced group**

| Group code | SEQ ID NO: | Sequence | Group code | SEQ ID NO: | Sequence | Group code | SEQ ID NO: | Sequence | Group code | SEQ ID NO: | Sequence |
|---|---|---|---|---|---|---|---|---|---|---|---|
| 1 | 1 | tcacattgct | 25 | 97 | gtatagctgc | 49 | 193 | agctactctg | 73 | 289 | agagacttac |
| | 2 | aatggcgctc | | 98 | cagacatctg | | 194 | caacgtgagt | | 290 | cttatggccg |
| | 3 | gtctcaatga | | 99 | agcggtggat | | 195 | tcgatgctaa | | 291 | gcctgacgtt |
| | 4 | cggatgcaag | | 100 | tctctcaaca | | 196 | gttgcaagcc | | 292 | tagcctaaga |
| 2 | 5 | tcgcttaagc | 26 | 101 | catccactgt | 50 | 197 | cgacatgtgt | 74 | 293 | aagcatatcc |
| | 6 | cgaggcttag | | 102 | gccgtgaaca | | 198 | gatgcgcata | | 294 | ctaatccgtt |
| | 7 | gtctaaggct | | 103 | ataagcggag | | 199 | tcgtgatcag | | 295 | gcttggtcga |
| | 8 | aatacgccta | | 104 | tggtattctc | | 200 | atcatcagcc | | 296 | tgcgcagaag |
| 3 | 9 | aagcctattg | 27 | 105 | acagttctca | 51 | 201 | tcaatggcgg | 75 | 297 | gatcctgata |
| | 10 | cgctactgca | | 106 | cattgagagc | | 202 | cagtaactct | | 298 | tcaagcacgg |
| | 11 | tcaagagcat | | 107 | gtcacgactg | | 203 | gttgcctgac | | 299 | ctgtagcgct |
| | 12 | gttgtgcagc | | 108 | tggcactgat | | 204 | agccgtaata | | 300 | agcgtattac |
| 4 | 13 | agacaggaat | 28 | 109 | aatgattcgc | 52 | 205 | ctaataggct | 76 | 301 | tgtctgattg |
| | 14 | ccttgccgta | | 110 | cgcttaagta | | 206 | tctcctccac | | 302 | accagacggt |
| | 15 | gtcattacgg | | 111 | ttgagcgacg | | 207 | agctgcatga | | 303 | gtgtctgacc |
| | 16 | taggcattcc | | 112 | gcaccgctat | | 208 | gaggagtatg | | 304 | caagactcaa |
| 5 | 17 | catatcatcg | 29 | 113 | acgaacggat | 53 | 209 | accacgtagc | 77 | 305 | tcctccacag |
| | 18 | gcacaacaat | | 114 | gaacggacta | | 210 | gaatgcagta | | 306 | agacgaggtc |
| | 19 | ttgtcgtggc | | 115 | tgctctctgg | | 211 | ttggtagcct | | 307 | ctggattact |
| | 20 | agcggtgcta | | 116 | cttgtatacc | | 212 | cgtcatctag | | 308 | gatatgctga |
| 6 | 21 | agccagtagg | 30 | 117 | caccagcaca | 54 | 213 | tcaatgaggt | 78 | 309 | ctggtcaagg |
| | 22 | gtaagtgtac | | 118 | ttgtctgtag | | 214 | agcgaagctg | | 310 | gctccattcc |
| | 23 | tagtcacgtt | | 119 | agtatcactc | | 215 | ctgtcttaac | | 311 | tgatgtcgaa |
| | 24 | cctgtcacca | | 120 | gcaggatggt | | 216 | gatcgcctca | | 312 | aacaaggctt |
| 7 | 25 | atcgtggatg | 31 | 121 | ttatccacgt | 55 | 217 | gttccgaatg | 79 | 313 | catctagaca |
| | 26 | tggagatcga | | 122 | acggttcgtc | | 218 | tacgtacgca | | 314 | gcggatacag |
| | 27 | cctcacagat | | 123 | gaccagtaag | | 219 | cggagttcac | | 315 | ttctgcttgt |
| | 28 | gaatctctcc | | 124 | cgtagagtca | | 220 | acatacgtgt | | 316 | agaacgcgtc |
| 8 | 29 | gcagactgac | 32 | 125 | acgttaaggt | 56 | 221 | ctgaagagat | 80 | 317 | aagacgaact |
| | 30 | ctcattaacg | | 126 | ctcagcttag | | 222 | gaagcctcca | | 318 | ccagtactgc |
| | 31 | tggtgagctt | | 127 | tgtgctccta | | 223 | tgtcttcatc | | 319 | gttcgctgta |
| | 32 | aatccgctga | | 128 | gaacaggacc | | 224 | acctgagtgg | | 320 | tgctatgcag |
| 9 | 33 | tcgcatcaac | 33 | 129 | gcgaccttga | 57 | 225 | gtacgtcctt | 81 | 321 | tacacgcgca |
| | 34 | agaacagtga | | 130 | atcgtgagtt | | 226 | aactaggtca | | 322 | aggtacgcag |
| | 35 | catgtctcct | | 131 | cgtcgtcaac | | 227 | tgtgtcaggc | | 323 | gttcgattgt |
| | 36 | gtctggagtg | | 132 | taataagccg | | 228 | ccgacataag | | 324 | ccagttaatc |
| 10 | 37 | gaggtctgtg | 34 | 133 | ccacttagta | 58 | 229 | tccacacgtc | 82 | 325 | taagatcgga |
| | 38 | ctatagacgt | | 134 | agtagctagt | | 230 | cggcacatga | | 326 | agctcggctt |
| | 39 | tgcagtgacc | | 135 | gacgaactcg | | 231 | gtatgttcct | | 327 | gttcgataag |
| | 40 | actccactaa | | 136 | ttgtcggcac | | 232 | aatgtggaag | | 328 | ccgatcatcc |
| 11 | 41 | gcgaagtagg | 35 | 137 | agcatatcgt | 59 | 233 | agacagacgt | 83 | 329 | actggactca |
| | 42 | tgcctaacct | | 138 | gtagacggag | | 234 | ttctgtggag | | 330 | caatagaggc |
| | 43 | aatggtctac | | 139 | cattctcatc | | 235 | caggtcttcc | | 331 | gtcacttaag |
| | 44 | ctatccggta | | 140 | tcgcggatca | | 236 | gctacacata | | 332 | tggctcgctt |
| 12 | 45 | tacgcttcag | 36 | 141 | ctggaggcaa | 60 | 237 | ctagcgacac | 84 | 333 | cgcactatgg |
| | 46 | cggagcatct | | 142 | gatacttgtg | | 238 | aggcattact | | 334 | gaggtacatt |
| | 47 | gtactagatc | | 143 | tgcctccact | | 239 | gacatccgga | | 335 | tcttagtgac |
| | 48 | acttagcgga | | 144 | acatgaatgc | | 240 | tcttgagttg | | 336 | atacgcgcca |
| 13 | 49 | atcactccat | 37 | 145 | tcagcagagg | 61 | 241 | acaacagaag | 85 | 337 | tgaggcatat |
| | 50 | gatcgcagtg | | 146 | ctgtgcatta | | 242 | cgcttgtgga | | 338 | attctatggc |
| | 51 | ccggaattcc | | 147 | agtaatcgac | | 243 | gttggtctcc | | 339 | ccgtagcatg |
| | 52 | tgattggaga | | 148 | gacctgtcct | | 244 | tagcacactt | | 340 | gacactgcca |
| 14 | 53 | cacaaggtcg | 38 | 149 | aagcggtgaa | 62 | 245 | gcttgcaata | 86 | 341 | acggcattaa |
| | 54 | tctcgcagga | | 150 | ctatcacact | | 246 | tggacgtgct | | 342 | gtaagcgagg |
| | 55 | gtggtatcat | | 151 | gccgttatgc | | 247 | aacgaaccgg | | 343 | cattatcgct |
| | 56 | agatctcatc | | 152 | tgtaacgctg | | 248 | ctacttgtac | | 344 | tgcctgactc |
| 15 | 57 | gatggagatt | 39 | 153 | gcgtgtaact | 63 | 249 | cggtgagtga | 87 | 345 | gactcatcca |
| | 58 | ctcattctgc | | 154 | catgtaccac | | 250 | gcaatgcatt | | 346 | acgaacatac |
| | 59 | tggcagacaa | | 155 | tgccactgta | | 251 | ttccattcac | | 347 | ttacgtcggt |
| | 60 | acatcctgcg | | 156 | ataacggtgg | | 252 | aatgccagcg | | 348 | cgtgtggatg |
| 16 | 61 | acgtcgcaga | 40 | 157 | cttgaaggtt | 64 | 253 | tactcttctc | 88 | 349 | cctaacattc |
| | 62 | tgtataggct | | 158 | gcctctatgg | | 254 | aggaaggtaa | | 350 | atcgcacgca |
| | 63 | caacacattg | | 159 | tagatccacc | | 255 | gttggacggt | | 351 | gaacgttcgg |
| | 64 | gtcggttcac | | 160 | agacggtcaa | | 256 | ccactcaacg | | 352 | tggttggaat |
| 17 | 65 | agccataagc | 41 | 161 | gctggattaa | 65 | 257 | aatgactggt | 89 | 353 | aacaagtgag |
| | 66 | gtattccgag | | 162 | taacaccggc | | 258 | gtgccacaac | | 354 | gttgttgctc |
| | 67 | cataggttca | | 163 | atgactgccg | | 259 | cgaatgatcg | | 355 | tgacgcaact |
| | 68 | tcggcagctt | | 164 | cgcttgaatt | | 260 | tcctgtgcta | | 356 | ccgtcactga |
| 18 | 69 | gttcggtcct | 42 | 165 | ctatagcgag | 66 | 261 | tgtgaattgg | 90 | 357 | tgttattccg |
| | 70 | tggattgtag | | 166 | aacgttgttc | | 262 | aaccggcctt | | 358 | ctactcaaga |
| | 71 | ccagaacgtc | | 167 | gctaccacgt | | 263 | ctatccgacc | | 359 | acgagacgtc |
| | 72 | aactccaaga | | 168 | tggcgataca | | 264 | gcgattagaa | | 360 | gacgcggtat |
| 19 | 73 | cgtacactgg | 43 | 169 | tccgccaatc | 67 | 265 | cgtaaccgca | 91 | 361 | gatgacgtta |
| | 74 | gcacacagca | | 170 | caactagtgt | | 266 | gactgataac | | 362 | agccgatacc |
| | 75 | atggtgtatc | | 171 | agtaagccaa | | 267 | atgcttactg | | 363 | ttatctcgag |
| | 76 | tactgtgcat | | 172 | gtgtgttgcg | | 268 | tcagcggtgt | | 364 | ccgatgacgt |
| 20 | 77 | cggcaatcag | 44 | 173 | gatcagatgg | 68 | 269 | acataacacc | 92 | 365 | gtgagttcgc |
| | 78 | gtagttcgga | | 174 | ctcgtaggtc | | 270 | cacctgaggt | | 366 | acacaacatg |
| | 79 | tacaggaact | | 175 | tcgtgtccat | | 271 | gtgactgtaa | | 367 | cgtgcgatca |
| | 80 | acttccgttc | | 176 | agaacctaca | | 272 | tgtggctctg | | 368 | tacttcggat |
| 21 | 81 | tgctccacga | 45 | 177 | ccgcattcct | 69 | 273 | ctcagactct | 93 | 369 | ctatcggtgt |
| | 82 | aatcaaggtc | | 178 | gttggacata | | 274 | acacatgcta | | 370 | gacattcaag |
| | 83 | gtaggtcaat | | 179 | tgatcgaggc | | 275 | tgtgcctaag | | 371 | agtgacacca |
| | 84 | ccgatgttcg | | 180 | aacatcgtag | | 276 | gagttgaggc | | 372 | tcgcgatgtc |
| 22 | 85 | gacgtgtgca | 46 | 181 | gttgcgcgaa | 70 | 277 | agccgttctc | 94 | 373 | cgagtcagtc |
| | 86 | tcgccacttc | | 182 | acaagtaagc | | 278 | ttgttggtct | | 374 | ttgccagtga |
| | 87 | ataagcacgt | | 183 | cgcttctcct | | 279 | caagacaaga | | 375 | aacaagcact |
| | 88 | cgttatgaag | | 184 | tagcaagttg | | 280 | gctacacgag | | 376 | gcttgttcag |
| 23 | 89 | aatagagcca | 47 | 185 | aggcctcttc | 71 | 281 | gtgacgcgat | 95 | 377 | tagtgatgtg |
| | 90 | gtacctcgac | | 186 | gtaatgtcgt | | 282 | aacctctctg | | 378 | cgtgtgacat |
| | 91 | ccggtgattg | | 187 | cactgcagag | | 283 | tcttgagaga | | 379 | atccaccacc |
| | 92 | tgctactagt | | 188 | tctgaagaca | | 284 | cgagatatcc | | 380 | gcaactgtga |
| 24 | 93 | gatccggact | 48 | 189 | gctaaggata | 72 | 285 | gaaggattca | 96 | 381 | atccaccggt |
| | 94 | ttaggcacaa | | 190 | cacggttggt | | 286 | tcgcctggtt | | 382 | ccgtcattac |
| | 95 | agcattcttg | | 191 | tgaccaccag | | 287 | agtaacacgg | | 383 | tgagtggcta |
| | 96 | ccgtaatggc | | 192 | atgttcatcc | | 288 | ctcttgcaac | | 384 | gatagtaacg |

**Table 2: 384 types of 8-balanced tag sequences**

| Group code | SEQ ID NO: | Sequence | Group code | SEQ ID NO: | Sequence | Group code | SEQ ID NO: | Sequence | Group code | SEQ ID NO: | Sequence |
|---|---|---|---|---|---|---|---|---|---|---|---|
| 1 | 385 | cgtcgatgac | 13 | 481 | gtcaactcgg | 25 | 577 | tcaatgaggt | 37 | 673 | tgacaatgac |
| | 386 | atataaggcg | | 482 | tggaagcaca | | 578 | gtccaagctg | | 674 | aatcacctct |
| | 387 | gatcgtgctc | | 483 | tcgttgtagc | | 579 | gcgcactaag | | 675 | aacagacctg |
| | 388 | cagtcttcgg | | 484 | agccgaagtt | | 580 | cggtgagtga | | 676 | gctagtgtgt |
| | 389 | agaacgatct | | 485 | gatgcaacct | | 581 | tgaatccgac | | 677 | gcggttgata |
| | 390 | ttggtgcatt | | 486 | acttccgttc | | 582 | aatgctcact | | 678 | ctagcgacac |
| | 391 | gccgtcataa | | 487 | caagttggaa | | 583 | atcggttcca | | 679 | cggttgaacg |
| | 392 | tccaaccaga | | 488 | ctacgtctag | | 584 | cattcgattc | | 680 | ttctcctgga |
| 2 | 393 | gatagcaaga | 14 | 489 | tgtcgttaag | 26 | 585 | ttatgcctac | 38 | 681 | agttagctgg |
| | 394 | accgtgcttc | | 490 | gtctcaatga | | 586 | atcctccgac | | 682 | agtcctgtaa |
| | 395 | gcagatgtaa | | 491 | gatcaagcca | | 587 | cgcagatata | | 683 | taaggccggt |
| | 396 | tgttggagcg | | 492 | aactccgatc | | 588 | acaacatgtg | | 684 | gccttatcct |
| | 397 | ttgtatccac | | 493 | ctaagtcttc | | 589 | tcggatgagg | | 685 | cagcattcaa |
| | 398 | cgcacagatg | | 494 | tgagagcgct | | 590 | cgtgcgatca | | 686 | tcgagcaatc |
| | 399 | caactctcgt | | 495 | acgatctcgg | | 591 | gatcttacgt | | 687 | gtaacgaacg |
| | 400 | atgccatgct | | 496 | ccggtgagat | | 592 | gagtaggcct | | 688 | ctcgtaggtc |
| 3 | 401 | aggcagctta | 15 | 497 | catctcatga | 27 | 593 | aagagagaag | 39 | 689 | ctggattact |
| | 402 | tagcctagcg | | 498 | ctgtgactcg | | 594 | gttctcacgg | | 690 | gtatgttcct |
| | 403 | atcacgtgcg | | 499 | agaccttgga | | 595 | aagcggtgaa | | 691 | tgttcgcgac |
| | 404 | cgttatgcgc | | 500 | actgtcgacg | | 596 | cgctcagtta | | 692 | cacggaattg |
| | 405 | caaggatcga | | 501 | gtgaagacac | | 597 | ctagacactc | | 693 | agcacaatgc |
| | 406 | gttgtcgtat | | 502 | tactgtgcat | | 598 | gcttattgct | | 694 | acacaccgga |
| | 407 | gcaatcaatc | | 503 | gcagagcgtt | | 599 | tgcgttctgc | | 695 | tatctcgcta |
| | 408 | tcctgacaat | | 504 | tgcacatatc | | 600 | tcaacgcact | | 696 | gcgatggaag |
| 4 | 409 | aggtgcctta | 16 | 505 | cctgtgtaac | 28 | 601 | cttgcttaca | 40 | 697 | taactcgtgg |
| | 410 | aagaaccaag | | 506 | aacgatgcca | | 602 | ctcctcgcaa | | 698 | acaaccagta |
| | 411 | catacatgac | | 507 | tcgagcatag | | 603 | tctcagcctc | | 699 | agcgtacgtc |
| | 412 | tgcctggtga | | 508 | gaattcctgt | | 604 | gacggagtac | | 700 | tcgactctgt |
| | 413 | tctcggagtt | | 509 | agaacgtccg | | 605 | acgtctatct | | 701 | ctgtatgcca |
| | 414 | gtcgtagact | | 510 | cgccaaggta | | 606 | gaatagagtg | | 702 | gacgagacct |
| | 415 | gcatataccg | | 511 | ttgtgaaggc | | 607 | aggatacggt | | 703 | cgttggtaac |
| | 416 | ctagcttcgc | | 512 | gttcctcatt | | 608 | tgaagctagg | | 704 | gttcgataag |
| 5 | 417 | gacgtatcaa | 17 | 513 | cgtcactatt | 29 | 609 | aacctcgcac | 41 | 705 | caagcgcgat |
| | 418 | cctgctagga | | 514 | gcgatgcaga | | 610 | gatccggact | | 706 | gtgtgagtgc |
| | 419 | caacttggcg | | 515 | cgtgtcctag | | 611 | gtgtctacag | | 707 | gcagcataat |
| | 420 | atgcgacctc | | 516 | gaagaatgga | | 612 | acggagaatc | | 708 | ttcttgtgca |
| | 421 | gttaaggacg | | 517 | atgtgtggct | | 613 | tgttacctca | | 709 | tggcacattg |
| | 422 | tccaagttgt | | 518 | tcctgtacac | | 614 | tcaatacgtg | | 710 | aacaatacgc |
| | 423 | aggtccattc | | 519 | ttaccgattc | | 615 | ctaagttggt | | 711 | cgtattgctg |
| | 424 | tgatgccaat | | 520 | aacacagccg | | 616 | cgcggattga | | 712 | actcgccaca |
| 6 | 425 | cctaagagtt | 18 | 521 | tcataccaag | 30 | 617 | acctcacata | 42 | 713 | gctgcttggt |
| | 426 | gatactagct | | 522 | gaagcttact | | 618 | cggactgtct | | 714 | ttgtggatac |
| | 427 | aaggcatctc | | 523 | gtcagtaggt | | 619 | cgtggcagaa | | 715 | accaaggcga |
| | 428 | tggcatctgg | | 524 | ctgtgcgtag | | 620 | attcgaatgc | | 716 | agaagaccta |
| | 429 | acactggagc | | 525 | agtgaatgga | | 621 | taggttcaag | | 717 | tagctctgtc |
| | 430 | gtatgccaca | | 526 | tccacggttc | | 622 | gtccactctc | | 718 | cgacttctat |
| | 431 | ctcttagtag | | 527 | aatctgcctc | | 623 | gaatagtgct | | 719 | ctctccaacg |
| | 432 | tgcggctcaa | | 528 | cggctaacca | | 624 | tcaatggcgg | | 720 | gatgaagacg |
| 7 | 433 | taaggctaga | 19 | 529 | gacatacagt | 31 | 625 | aagcatcctg | 43 | 721 | tccagctcat |
| | 434 | gaggagataa | | 530 | agaggcctca | | 626 | gtggtgttca | | 722 | accaagactc |
| | 435 | cgttagcact | | 531 | cctgatattg | | 627 | gtacaacgtt | | 723 | caactgcgca |
| | 436 | aggctaggat | | 532 | gtgctgaact | | 628 | cctgcagcat | | 724 | agtggatatc |
| | 437 | atctcactgg | | 533 | cgatggtcag | | 629 | tctatcgtac | | 725 | gtactaagag |
| | 438 | tccagttctc | | 534 | aatacagcgc | | 630 | cactcgtagc | | 726 | gtgtacgtcg |
| | 439 | gtaaccgctc | | 535 | tcgtcctgac | | 631 | tgcagcagca | | 727 | cggtctctgt |
| | 440 | cctcttagcg | | 536 | ttccatggta | | 632 | agatgtaagg | | 728 | tatgctgaga |
| 8 | 441 | actaaggctg | 20 | 537 | cgttcgactg | 32 | 633 | gtgtaaccgc | 44 | 729 | aataggtagg |
| | 442 | gaggagtatg | | 538 | cgctacactc | | 634 | catcggaaga | | 730 | agcttgcgct |
| | 443 | tagtcaacgc | | 539 | atagatcggt | | 635 | gaggaattac | | 731 | tggagccgat |
| | 444 | ccatcaagga | | 540 | accagattca | | 636 | tgttcgctct | | 732 | ccgcatacta |
| | 445 | agcctctgct | | 541 | gatacggagg | | 637 | accgtctata | | 733 | ttcgatgacg |
| | 446 | ttcgttcaca | | 542 | ttaggaggaa | | 638 | tgcctcagag | | 734 | gatccaatac |
| | 447 | gtaagtgtac | | 543 | gcgcttctac | | 639 | acaagtgccg | | 735 | gcattctcgc |
| | 448 | cgtcgcctat | | 544 | tagctctact | | 640 | ctaactggtt | | 736 | ctagcagtta |
| 9 | 449 | tgtgaaggag | 21 | 545 | ccacctgctt | 33 | 641 | cagaacgtgg | 45 | 737 | cgttgacgct |
| | 450 | agaccggttc | | 546 | cgtaggtctg | | 642 | gttcttctgt | | 738 | agtatatgcg |
| | 451 | ctcgcctaac | | 547 | tcttagtgac | | 643 | cggtgaagtc | | 739 | acctccgcta |
| | 452 | ccgctgtcta | | 548 | atcctagaac | | 644 | gtaagatgag | | 740 | caacacgaat |
| | 453 | gacataacct | | 549 | aggtgtaggt | | 645 | tgccatcaca | | 741 | ttgagtaagg |
| | 454 | gagaatagca | | 550 | gtaaccatca | | 646 | tcctcggata | | 742 | gaagctctta |
| | 455 | ttatgtcagg | | 551 | taggtccaga | | 647 | acagtgtcct | | 743 | tccgagatgc |
| | 456 | acttgcctgt | | 552 | gacgaactcg | | 648 | aatgccacac | | 744 | gtgctgtcac |
| 10 | 457 | tgctggatct | 22 | 553 | ccaacagatt | 34 | 649 | gaccactcga | 46 | 745 | agcttccagc |
| | 458 | agtagtgttg | | 554 | cctctatctc | | 650 | atggacaaca | | 746 | tgcctatcgc |
| | 459 | ctccaatcct | | 555 | gagtgtctca | | 651 | aacctacggt | | 747 | caatctcgcg |
| | 460 | cctatgtgta | | 556 | ttggcttaag | | 652 | tctaggattg | | 748 | cttcctacaa |
| | 461 | taatatccgc | | 557 | agtcgcatgg | | 653 | cggattctcg | | 749 | acgaggtact |
| | 462 | gtgccacaac | | 558 | atcttgcggc | | 654 | cgatcttctc | | 750 | gatgaaggat |
| | 463 | gcagtcagga | | 559 | tgcaaggcct | | 655 | tcatcggaat | | 751 | tcgagcgtta |
| | 464 | aaggccgaag | | 560 | gaagacagaa | | 656 | gttggaggac | | 752 | gtagagattg |
| 11 | 465 | cgtgttagag | 23 | 561 | acagactcat | 35 | 657 | acacatgcta | 47 | 753 | acggctagag |
| | 466 | acaggacgat | | 562 | gagttgaggc | | 658 | ccttaggacg | | 754 | gctatagctt |
| | 467 | cggataacgg | | 563 | gttctagacg | | 659 | ctaaccaatc | | 755 | tgcgtcatgg |
| | 468 | gttagtgcct | | 564 | agttcaggcg | | 660 | aacgcacgag | | 756 | ttccaacatc |
| | 469 | aagcacgaca | | 565 | cagacgatga | | 661 | gtcggttcac | | 757 | cggtgttgga |
| | 470 | gtctccttgc | | 566 | tccaacctat | | 662 | tggctcatgt | | 758 | gattgcgcct |
| | 471 | tcaccgtata | | 567 | ttacgttctc | | 663 | tggtggttgt | | 759 | ataacgctca |
| | 472 | tactagcttc | | 568 | cgcggtcata | | 664 | gatatacgca | | 760 | caacagtaac |
| 12 | 473 | accattcacg | 24 | 569 | aatccttccg | 36 | 665 | cacgaacact | 48 | 761 | actggaggag |
| | 474 | agtagctagt | | 570 | gaggattgaa | | 666 | tgcgtgagca | | 762 | gccggtaagt |
| | 475 | ttccgaaggt | | 571 | atcggagtgc | | 667 | gtactgacga | | 763 | ctcacactta |
| | 476 | gtaccaacca | | 572 | gcgttaagtg | | 668 | ccgtgagttc | | 764 | agacagtggc |
| | 477 | tatgtgtgtc | | 573 | cctcggcaat | | 669 | acttacttgg | | 765 | ctgatgtcct |
| | 478 | cgatcggtac | | 574 | ttaacggctt | | 670 | agaagctcat | | 766 | gagttcacaa |
| | 479 | gaggatctag | | 575 | tgattccaca | | 671 | gttactggac | | 767 | tgttctcttc |
| | 480 | ccgtacgcta | | 576 | cgcaacatgc | | 672 | tagcctcatg | | 768 | taacacgacg |

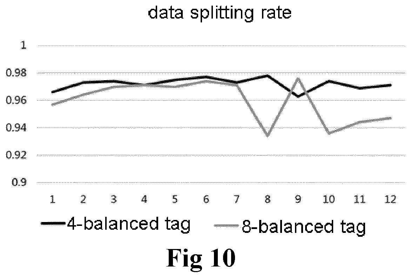

The splitting rate of sequencing data in the 4-balanced tags groups will be higher, because the sequencing machine reads the bases with the balanced composition more accurately, and the unbalanced bases will cause reading errors and reduce the splitting rate of the sequencing data. When 12 samples are pooled in equal proportions, the 4-balanced tags and 8-balanced tags were both used to construct libraries for sequencing. From the results as shown in Figure 10, for the 4-balanced tags, the 12 samples have almost the same sequencing data splitting. For the 8-balanced tags, some samples of the 12 samples have significantly reduced data splitting.

Based on the above research results, the inventors proposed the technical solutions of the invention.

In a typical mode of the invention, a dual library tags composition is provided. The dual library tags composition includes a plurality of 5' end library tags and a plurality of 3' end library tags. The lengths of the 5' end library tags are all the same, the lengths of the 3' end library tags are all the same and the occurrences of each base at the same position in the dual library tags composition are also the same.

In the dual library tags composition provided by the invention, by controlling the lengths of the 5' end library tags are all the same, the lengths of the 3' end library tags are all the same, and occurrences of each base at the same position are all the same, multiple libraries with good- base balanced dual tags can be obtained. When the multiple libraries are pooled for sequencing, the dual tags sequence can be read more accurately, and the sequencing data can be split more effectively.

In order to further improve the base balance and reading accuracy of the library tags, in a preferred embodiment, the lengths of the 5' end library tags are the same with the lengths of the 3' end library tags, preferably is any fixed length between 6-10bp. The lengths of the library tags at both ends are the same, so that when the samples are split, the same number of bases in the library tags at both ends participates in determining the source of the sample, so the probability of support provided by the libraries from both ends is the same. It can avoid that one end of the library tag is longer and the reference probability of support is higher, and the other end of the library tag is shorter, and the reference probability of support is lower, which leads to the result that is more biased to rely on tags on one end.

Preferably, in the dual library tags composition, there are at least 3 base differences between any two library tags, and the number of continuous same bases in any library tag does not exceed 3, preferably, GC contents in all library tags are all 40-60%. When library tags meet the above base optimization principles and are used in combination, the base balance is better, the reading results are accurate, and the data splitting rate is also higher.

Preferably, the dual library tags composition includes a composition of 4-balanced dual library tags, or a composition of 8-balanced dual library tags, the combination of 4-balanced dual library tags includes 4n 5' end library tags and 4n 3' end library tags. The combination of 8-balanced dual library tags includes 8n 5' end library tags and 8n 3' end library tags, n is an integer greater than or equal to 1.

In a preferred embodiment, in the composition of 4-balanced dual library tags, 5' end library tags are selected from any one of the 96 groups shown in Table 1, and the 3' end library tags are selected from any one of the 96 groups shown in Table 1 that is different from the 5' end library tag group.

In a preferred embodiment, in the composition of 8-balanced dual library tags, 5' end library tags are selected from any one of the 48 groups shown in Table 2, and the 3' end library tags are selected from any one of the 48 groups shown in Table 2 that is different from the 5' end library tag group.

In the second typical mode of the invention, a composition of amplification primers with dual library tags based on MGI sequencing platform is provided, and the composition of amplification primers includes a plurality of amplification primer pairs with dual library tags, each amplification primer pair includes a 5' end library tag and a 3' end library tag, and the lengths of the 5' end library tags are all the same and the lengths of the 3' end library tags of the amplification primer pairs are all the same, and the occurrences of each base at the same position are also all the same.

By controlling the lengths of 5' end library tags are all the same and the lengths of the 3' end library tags of the plurality of amplification primer pairs are all the same, and the occurrences of each base at the same position are also all the same, when the dual tags in the composition of amplification primers are used to label multiple pooled samples for sequencing, the reading of the tag bases is balanced, the results are more accurate, and the samples data split according to the tags are also more accurate, which improves the splitting rate of the sequencing data.

Based on the same lengths of the 5' end library tags and the same lengths of the 3' end library tags of the above pooled samples, in order to further improve the base balance and reading accuracy of the library tags, in a preferred embodiment, the lengths of the 5' end library tags and the lengths of the 3' end library tags of the plurality of amplification primer pairs are the same. The lengths of the library tags at both ends of each pair of amplification primers are the same, so that when the samples are split, the same number of bases in the library tags at both ends participates in determining the source of the sample, and the probability of support provided by the libraries at both ends is the same. It can avoid that one end of the library tag is longer and the reference probability of support is higher, and the other end of the library tag is shorter, and the reference probability of support is lower, which leads to the result that is more biased to rely on tags on one end.

More preferably, the lengths of 5' end library tags and the 3' end library tags are both any fixed length between 6-10bp, further the preferred length is 10bp, which has greater discrimination and more beneficial effects than other lengths such as 6bp or 8bp.

In order to provide more balanced library tags, in a preferred embodiment, in the composition of amplification primers, there are at least 3 base differences between any two library tags, and the number of the continuous same base in any one of the library tags does not exceed 3, and the GC contents of the library tags are all 40-60%. When library tags meet the above base optimization principle and are used in combination, the balance of base reading is better, the result is more accurate, and the splitting rate of the sequencing data is also higher.

In a preferred embodiment, the mentioned composition of amplification primers includes a combination of 4n 4-balanced tags amplification primer pairs, or a combination of 8n 8-balanced tags amplification primer pairs, where n is an integer greater than or equal to 1. More preferably, in the 4n 4-balanced tags amplification primer pairs, the 5' end library tags are selected from any one or more of the 96 groups shown in Table 1, and the 3' end library tags are selected from any one or more of the 96 groups different from the 5'-end library tags shown in Table 1. The number of groups here is determined according to the actual needs. The combinations of 96 groups of tag sequences in Table 1 makes higher reading accuracy, so sequencing data splitting is more accurate, and the splitting rate is also higher.

In another preferred embodiment, in the 8n amplification primer pairs with 8-balanced tags, the 5' end library tags are selected from any one or more of the 48 groups shown in Table 2, and the 3' end library tags are selected from any one or more of the 48 groups shown in Table 2 that are different from the 5' end of the library tag groups.

In the above composition of amplification primers, each amplification primer pair further includes a 5' end universal amplification sequence and a 3' end universal amplification sequence, and the 5'-end universal amplification sequence includes the universal downstream sequence of the 5' end library tags and the universal upstream sequence of the 5' end library tags, the 3' end universal amplification sequence includes the universal downstream sequence of the 3' end library tags and the universal upstream sequence of the 3' end library tags. The specific sequence of the universal amplification sequence in each amplification primer pair is determined according to the existing universal sequences of MGI sequencing platform. The combination of amplification primers formed by the amplification primer pairs containing the above library tags can improve the reading accuracy of the library tags when the samples are pooled and sequenced on the machine, thereby improving the accuracy of the sequencing data of each sample.

As mentioned above, the library construction can adopt a relatively short bubble adapter (that is the number of unpaired bases in the middle region is 30±5bp), or a relatively long bubble adapter (the number of unpaired bases in the middle region is 45±5bp). Correspondingly, the universal sequence in the amplification primer pair here can also be adjusted to a longer or shorter universal amplification sequence according to the length of the bubble adapter.

In a preferred embodiment, corresponding to the use of a shorter bubble adapter, the universal upstream sequence of the 5' end library tag is SEQ ID NO: 793, and the universal downstream sequence of the 5' end library tag is SEQ. ID NO: 794; the universal upstream sequence of the 3' end library tag is SEQ ID NO: 795, and the universal downstream sequence of the 3' end library tag is SEQ ID NO: 796.

In another preferred embodiment, corresponding to the use of a longer bubble adapter, the universal upstream sequence of the 5' end library tag is SEQ ID NO: 793, and the universal downstream sequence of the 5' end library tag is SEQ. ID NO: 797; the universal upstream sequence of the 3' end library tag is SEQ ID NO: 795, and the universal downstream sequence of the 3' end library tag is SEQ ID NO: 798.

In the third mode of the invention, a library construct kit based on MGI sequencing platform is also provided, the kit includes any one the composition of amplification primers mentioned above. The dual library tags in the amplification primers have the base balance, so the tag sequences of each sample after the sequencing can be accurately read, and the data split accuracy of the pooled samples are improved.

In order to further improve the convenience of the library construction, the kit may further includes a bubble adapter of the MGI sequencing platform, the bubble adapter includes a first adapter sequence and a second adapter sequence, and the first adapter sequence is SEQ ID NO: 769, the second adapter sequence is SEQ ID NO: 770, or the first adapter sequence is SEQ ID NO: 773, the second adapter sequence is SEQ ID NO: 774. Compared to a relatively longer bubble adapter, the shorter bubble adapter can not only improve the stability of the ligation and have higher ligation efficiency, but also is more compatible in the subsequent PCR amplification procedures after the adapter ligation.

In the fourth embodiment of the invention, a method of constructing a sequencing library applying any of the above kits based on MGI sequencing platform is provided. When the libraries constructed as the above kits are sequenced on the machine, the balance of the library tags is better, and the reading accuracy data splitting rate are higher.

In the fifth embodiment of the invention, a sequencing library is also provided. The sequencing library includes any of the composition of amplification primers, or is constructed through any of the above methods. The balance of the library tags in the sequencing library is better, and the read accuracy of the library tags after sequencing is higher, and the data splitting rate is higher.

The advantages of the invention will be further described below in the embodiment. It should be noted that the following examples uses NadPrep^{™} DNA library prep kit to construct the libraries.

Item No.: 1002212 NadPrep^{®} Plasma Free DNA dual tag library prep kit (for MGI).

Item No.: 1003811 User's Guide V1. 0 (Nanodigmbio,Nanjing).

The process is briefly described as follows.

DNA Sample Fragment --- End Repair and A-Tailing --- Ligation --- Fragment Selection --- PCR Amplification --- Library Purification, Quantitative and Quality Control --- Sequencing or targeting Sequencing on MGI platform.

It will also be noted that the following examples are merely exemplary, and do not limit the method of the invention to be the following mtheods.

### Example 1 Library Prep Solution 1 and Solution 2

### Steps: Refer to NadPrep^{™} DNA library prep kit (for MGI) (201909Version2.0)

The differences lie in the bubble adapter sequence and the amplification primer sequence.

### (1)Solution1:

Bubble adapter sequence:
SEQ ID NO:769 (adapter 1) and SEQ ID NO:770 (adapter 2):
SEQ ID NO:769: (31bp) /phos/agtcggaggccaagcggtcttaggaagacaa;
SEQ ID NO:770(40bp): ttgtcttcctaacaggaacgacatggctacgatccgact*t.
SEQ ID NO:771 (amplification primer 1) 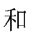 SEQ ID NO:772 (amplification primer 2):
SEQ ID NO:771: (64bp)
   /phos/ctctcagtacgtcagcagttnnnnnnnnnncaactccttggctcacagaacgacatggctacga, wherein the sequence before nnnnnnnnnn (/phos/ctctcagtacgtcagcagtt) is SEQ ID NO: 793, the sequence after nnnnnnnnnn (caactccttggctcacagaac**gacatggctacga**) is SEQ ID NO: 794, (**gacatggctacga** is the prolonged part compared to the solution 2).
SEQ ID NO:772: (52bp)
   gcatggcgaccttatcagnnnnnnnnnnttgtcttcctaagaccgcttggcc, wherein the sequence before nnnnnnnnnn (gcatggcgaccttatcag) is SEQ ID NO:795, the sequence after nnnnnnnnnn (ttgtcttcctaagaccgcttgg**cc**) is SEQ ID NO:796 (**cc** is the prolonged part compared to the solution 2).

### Characteristics of solution 1:

1. The complementary portion of the adapter is 7 + 13bp (belong to the region of 20 ± 2bp), the bubble structure region is 20 + 12bp (belong to the region of 30 + 5bp);
2. The amplification primer is a little longer.

### Advantages:

1. The bubble structure is shorter, so the annealed structure is stable.
2. The amplification primer is compatible to single-end amplification primers and single-end tags (see the CN application NO. 201910229527.4).

### (2)Solution2:

Adapter sequence
SEQ ID NO: 773 (adapter 1) 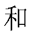 SEQ ID NO: 774 (adapter 2).
SEQ ID NO: 773(35bp): /phos/agtcggaggccaagcggtcttaggaagacaatcag.
SEQ ID NO: 774(59bp):
   ctgattgtcttcctaagcaactccttggctcacagaacgacatggctacgatccgactt.
SEQ ID NO:775 (amplification primer 1) 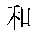 SEQ ID NO:776 (amplification primer 2).
SEQ ID NO:775: (51bp)
   /phos/ctctcagtacgtcagcagttnnnnnnnnnncaactccttggctcacagaac; wherein the sequence before nnnnnnnnnn (/phos/CTCtcagtacgtcagcagtt) is SEQ ID NO:793, the sequence after nnnnnnnnnn (caactccttggctcacagaac) is SEQ ID NO:797.
SEQ ID NO:776: (50bp)
   gcatggcgaccttatcagnnnnnnnnnnttgtcttcctaagaccgcttgg, wherein the sequence before nnnnnnnnnn (gcatggcgaccttatcag) is SEQ ID NO:795, the sequence after nnnnnnnnnn (ttgtcttcctaagaccgcttgg) is SEQ ID NO:798.

Characteristics of solution 2:
1.The complementary portion of the adapter is 7+17bp (belong to the region of 25±2bp), the bubble structure is 34+12bp (belong to the region of 45±5bp);
2. The amplification primer is shorter.

Disadvantages compared with the solution1 :
1. The bubble structure is longer, so the annealed structure is relatively unstable.
2. The amplification primer is not compatible to other solutions (amplification primer is shorter, and there is no repeat sequence with the bubble structure).

The results of the adapter structures and amplification primers of the solution 1 and solution 2 are shown in Figure 4. The libraries with dual tags for MGI sequencing can both be obtained. 25 ng and 100 ng DNA are input for library construction in experiment process. The information is shown in the table below.

**Table3: Library yields from solution 1 and solution 2**

| Solution | DNA Input | PCR cycles | Library yield |
|---|---|---|---|
| 1 | 25 ng | 7 | 1222ng |
| | 100 ng | 5 | 1367 ng |
| 2 | 25 ng | 7 | 1176 ng |
| | 100 ng | 5 | 1159 ng |

The libraries with dual tags for MGI can both be obtained from solution 1 and solution 2, and the library yields are similar, as shown in Figure 9. But the solution 2 is not compatible to the single-end amplification primers and adapters with single-end tags.

### Example 2 Comparison of data splitting in 12 pooled samples between 4-balanced tags and 8-balanced tags

The solution using dual tags can effectively solve the crosstalk problems between samples (also called the tag jumping). But only when both ends of the tags are correct, the sequencing data can be effectively split. So the dual tags balance requirements are more stringent than the single-end tags. The present invention optimizes two set of solutions with 4-balanced tags and 8-balanced tags. This example adopted both 4-balanced tags and 8-balanced tags, and pooled 12 libraries for sequencing to detect splitting rate of each sample in two set of solutions. The experimental steps and information are as follows:
Steps: Refer to NadPrep^{™} DNA library prep kit (for MGI) (201909Version2.0) instructions. The only difference lies in that the adapter with single-end tags was changed into the adapter with dual tags.

The 4-balanced tags sequence used in the experiment is shown in Table 4, adjacent 4 tags are a group of balance, and each group is distinguished with bold or non-thickened fonts. The tag 1 is a forward arrangement of 384 tag sequences, and the tag 2 is a reverse arrangement of 384 tag sequences. The primer1 with tag 1 and the primer 2 with the tag 384 constitute the combination of the first group of dual tag primers. The primer1 with tag 2 and the primer 2 with the tag 383 constitute the combination of the second group of dual tag primers. Totally there will be 384 combinations.

8-balanced tags arrangements and 4-balanced tags arrangements are the same. The only difference is 8 tags in a group, as shown in Table 5. When 12 library tags are put together, the first 8 is balanced, the last 4 is unbalanced. For the 4-balanced tags combination, the 12 library tags are exactly balanced.

**Table 4: The 12 4-balanced tags combinations**

| Combination No. | Tag 1 No. | Tag 1 SEQ | Tag 2 No. | Tag 2 SEQ |
|---|---|---|---|---|
| XDI001 | 1(SEQ ID NO:1) | tcacattgct | 384(SEQ ID NO:384) | gatagtaacg |
| XDI002 | 2(SEQ ID NO:2) | aatggcgctc | 383(SEQ ID NO:383) | tgagtggcta |
| XDI003 | 3(SEQ ID NO:3) | gtctcaatga | 382(SEQ ID NO:382) | ccgtcattac |
| XDI004 | 4(SEQ ID NO:4) | cggatgcaag | 381(SEQ ID NO:381) | atccaccggt |
| **XDI005** | **5(SEQ ID NO:5)** | **tcgcttaagc** | **380(SEQ ID NO:380)** | **gcaactgtga** |
| **XDI006** | **6(SEQ ID NO:6)** | **cgaggcttag** | **379(SEQ ID NO:379)** | **atccaccacc** |
| **XDI007** | **7(SEQ ID NO:7)** | **gtctaaggct** | **378(SEQ ID NO:378)** | **cgtgtgacat** |
| **XDI008** | **8(SEQ ID NO:8)** | **aatacgccta** | **377(SEQ ID NO:377)** | **tagtgatgtg** |
| XDI009 | 9(SEQ ID NO:9) | aagcctattg | 376(SEQ ID NO:376) | gcttgttcag |
| XDI010 | 10(SEQ ID NO:10) | cgctactgca | 375(SEQ ID NO:375) | aacaagcact |
| XDI011 | 11(SEQ ID NO:11) | tcaagagcat | 374(SEQ ID NO:374) | ttgccagtga |
| XDI012 | 12(SEQ ID NO:12) | gttgtgcagc | 373(SEQ ID NO:373) | cgagtcagtc |

**Table5: The 12 4-balanced tags combinations**

| Combination No. | Tag 1 No. | Tag 1 SEQ | Tag 2 No. | Tag 2 SEQ |
|---|---|---|---|---|
| **MDI001** | **1(SEQ ID NO:385)** | **cgtcgatgac** | **384(SEQ ID NO:768)** | **taacacgacg** |
| **MDI002** | **2(SEQ ID NO:386)** | **atataaggcg** | **383(SEQ ID NO:767)** | **tgttctcttc** |
| **MDI003** | **3(SEQ ID NO:387)** | **gatcgtgctc** | **382(SEQ ID NO:766)** | **gagttcacaa** |
| **MDI004** | **4(SEQ ID NO:388)** | **cagtcttcgg** | **381(SEQ ID NO:765)** | **ctgatgtcct** |
| **MDI005** | **5(SEQ ID NO:389)** | **agaacgatct** | **380(SEQ ID NO:764)** | **agacagtggc** |
| **MDI006** | **6(SEQ ID NO:390)** | **ttggtgcatt** | **379(SEQ ID NO:763)** | **ctcacactta** |
| **MDI007** | **7(SEQ ID NO:391)** | **gccgtcataa** | **378(SEQ ID NO:762)** | **gccggtaagt** |
| **MDI008** | **8(SEQ ID NO:392)** | **tccaaccaga** | **377(SEQ ID NO:761)** | **actggaggag** |
| MDI009 | 9(SEQ ID NO:393) | gatagcaaga | 376(SEQ ID NO:760) | caacagtaac |
| MDI010 | 10(SEQ ID NO:394) | accgtgcttc | 375(SEQ ID NO:759) | ataacgctca |
| MDI011 | 11(SEQ ID NO:395) | gcagatgtaa | 374(SEQ ID NO:758) | gattgcgcct |
| MDI012 | 12(SEQ ID NO:396) | tgttggagcg | 373(SEQ ID NO:757) | cggtgttgga |

For the human genomic DNA standard, libraries are constructed with 12 combinations of dual 4-balanced tags and 8-balanced tags. The dual 4-balanced tags sequences are shown in Table 4, and the dual 8-balanced tags sequences are shown in Table 5. The 4-balanced libraries and 8-balanced libraries are sequenced and analyzed on MGI sequencing platform.

The two groups of libraries were splitting for two rounds, in the first round, the maximum fault tolerance (will split the sequencing error) was used for splitting, and in the second round, only one fault tolerance per tag was allowed for splitting. The results of data splitting were shown in Fig 10, the data splitting rate of the 12 libraries with 4-balanced tags is more stable, and the data splitting rate of the 12 libraries with 8-balanced tags is not stable. The results show that the balanced dual tags are more conducive to the effective data splitting of the MGI sequencer, herein the design of 8-balanced tags improves the data effective splitting rate to some extent, and the design of 4-balanced tags is better.

### Example 3

To ensure the performance difference between 48 groups of 8-balanced tags combinations provided by the present invention and the 12 groups of 8-balanced tags combinations provided by MGI manufacturing, the compatibility was considered when they were designed. There are 3 bases difference in any two sequences between 48 groups of 8-balanced tags combinations and 12 groups of 8-balanced tags combinations provided by MGI manufacturing.

In addition, there are other major distinguishes as follows:
1. The base composition of the tag sequence in the present invention is more equalized, and the GC content is 40% -60%, but the GC content of tags from MGI manufacturing is from 20% to 80%.
2. The tag sequence of the present invention performs a matching property of the adapter sequence of the solution 1 to ensure amplified libraries to be evenly produced. But some sequences from MGI manufacturing are not satisfied with the balanced requirement on library amplification efficiency.

In order to further verify the performance difference in amplification balance, a group of 8-balanced tags combinations MDI001-MDI008 of the invention and a group of 8-balanced tags combinations MGI001-MGI008 from MGI manufacturing (shown in Table 6) were selected to construct libraries: 100 ng of DNA as input, PCR amplification for 5 cycles to detect the library yields, and the results were shown in Table 7.

As shown in Table 7, the library yields from the invention are equal, while one library yield from MGI manufacturing is less than half of the normal value, which indicates that the optimized tag sequences of the present invention has better balance. Further, amplification efficiency is more stable. At the same time, due to the high throughput of the MGI sequencer, the two groups of 384 tags in the present invention are better than the 120 tags from MGI manufacturing to meet the throughput demand for pooled sequencing.

**Table 6: 8 combinations of 8-balanced tags from MGI manufacturing**

| Combination No. | Tag 1 No. | Tag 1 SEQ | Tag 2 No. | Tag 2 SEQ |
|---|---|---|---|---|
| **MGI001** | **1(SEQ ID NO:777)** | atgcatctaa | **120(SEQ ID NO:785)** | tagaggacaa |
| **MGI002** | **2(SEQ ID NO:778)** | agctctggac | **119(SEQ ID NO:786)** | cctagcgaat |
| **MGI003** | **3(SEQ ID NO:779)** | ctatcacgtg | **118(SEQ ID NO:787)** | gtagtcatcg |
| **MGI004** | **4(SEQ ID NO:780)** | ggactagtgg | **117(SEQ ID NO:788)** | gctgagctgt |
| **MGI005** | **5(SEQ ID NO:781)** | gccaagtcca | **116(SEQ ID NO:789)** | aacctagata |
| **MGI006** | **6(SEQ ID NO:782)** | cctgtcaagc | **115(SEQ ID NO:790)** | ttgccatctc |
| **MGI007** | **7(SEQ ID NO:783)** | tagaggtctt | **114(SEQ ID NO:791)** | agatcttgcg |
| **MGI008** | **8(SEQ ID NO:784)** | tatggcaact | **113(SEQ ID NO:792)** | cgctatcggc |

**Table 7**

| Library No. | Library Yield | Library No. | Library Yield |
|---|---|---|---|
| MGI001 | 1328 | MDI001 | 1386 |
| MGI002 | 1251 | MDI002 | 1255 |
| MGI003 | 1196 | MDI003 | 1229 |
| MGI004 | 1267 | MDI004 | 1311 |
| MGI005 | 667 | MDI005 | 1307 |
| MGI006 | 1345 | MDI006 | 1238 |
| MGI007 | 1257 | MDI007 | 1233 |
| MGI008 | 1344 | MDI008 | 1274 |

From the above embodiments, in the present invention dual library tags are introduced on MGI sequencing platform to solve the samples crosstalk problems caused by the synthesis, the experimental process, and the sequencing process, which will make the detection results more accurate. Furthermore, the inventors found that through test and optimization, when the middle structure of the bubble adapter is 30 ± 5bp, the paired base is 20 ± 2bp, the annealing of the bubble adaptors is most stable. Meanwhile, the amplification primer is an extended amplification primer, which can be compatible with the amplicons with single-end tags and adapters with molecular single-end tags. The bubble adapters with such a compositional structure are used together with the extended amplification primers in the library construction, which can be compatible with the existing single-end tags solution of MGI platform, and is convenient for the MGI sequencing application.

Based on the above, in order to obtain a better data splitting, the present invention optimized 384 combinations of 4-balanced tags and 8-balanced tags sequences, respectively, which provides optimal solution for high-throughput sequencing and sequencing data splitting for MGI platform.

## Claims

1. A sequencing library construction kit, comprising a composition of amplification primers with dual library tags based on MGI sequencing platform,
wherein the composition of amplification primers comprises a plurality of amplification primer pairs with dual library tags, each amplification primer pair comprises a 5' end library tag and a 3' end library tag,
wherein the lengths of multiple 5' end library tags of the amplification primer pairs are all the same, and the lengths of multiple 3' end library tags of the amplification primer pairs are all the same, and the occurrences of each base at the same position are also all the same,
wherein in the composition, there are at least 3 base differences between any two library tags, and the number of continuous same bases in any library tag does not exceed 3, GC contents in all library tags are all 40-60%,
wherein the composition comprises a combination of 4n 4-balanced amplification primer pairs, or a combination of 8n 8-balanced amplification primer pairs, wherein n is an integer greater than or equal to 1,
wherein in the combination of 4n 4-balanced amplification primer pairs, the 5' end library tags are selected from any one or more of the 96 groups shown in Table 1, and the 3' end library tags are selected from any one or more of the 96 groups shown in Table 1 that are different from the 5'-end library tags;
wherein in the combination of 8n 8-balanced amplification primer pairs, the 5' end library tags are selected from any one or more of the 48 groups shown in Table 2, and the 3' end library tags are selected from any one or more of the 48 groups shown in Table 2 that are different from the 5'-end library tags;
wherein each amplification primer pair further comprises a 5' end universal amplification sequence and a 3' end universal amplification sequence, the 5' end universal amplification sequence comprises an universal upstream sequence of the 5' end library tag and an universal downstream sequence of the 5' end library tag, and the 3' end universal amplification sequence comprises an universal upstream sequence of the 3' end library tag and an universal downstream sequence of the 3' end library tag;
wherein the universal upstream sequence of the 5' end library tag is SEQ ID NO: 793, and the universal downstream sequence of the 5' end library tags is SEQ ID NO: 794; the universal upstream sequence of the 3' end library tag is SEQ ID NO: 795, and the universal downstream sequence of the 3' end library tag is SEQ ID NO: 796; or
the universal upstream sequence of the 5' end library tag is SEQ ID NO: 793, and the universal downstream sequence of the 5' end library tag is SEQ ID NO: 797; the universal upstream sequence of the 3' end library tag is SEQ ID NO: 795, and the universal downstream sequence of the 3' end library tag is SEQ ID NO: 798;
wherein the kit further comprises bubble adapters, wherein the bubble adapters comprise a first adapter sequence and a second adapter sequence, the first adapter sequence is SEQ ID NO: 769, and the second adapter sequence is SEQ ID NO: 770, which are used together with the amplification primer pair comprising the SEQ ID NO: 793, SEQ ID NO: 794, SEQ ID NO: 795 and SEQ ID NO: 796;
or
the first adapter sequence is SEQ ID NO: 773, and the second adapter sequence is SEQ ID NO: 774, which are used together with the amplification primer pair comprising the SEQ ID NO: 793, SEQ ID NO: 797, SEQ ID NO: 795 and SEQ ID NO: 798.

2. A method for constructing a sequencing library based on MGI sequencing platform, comprising applying the kit as claimed in claim 1 to construct a library.

3. A sequencing library, comprising any one of the combination of amplification primers and the bubble adapters in the kit as claimed in claim 1.

## Patentansprüche

1. Sequenzierungsbibliothek-Konstruktionskit,
umfassend eine Zusammensetzung von Amplifikationsprimern mit dualen Bibliotheksmarkierungen, basierend auf einer MGI-Sequenzierungsplattform,
wobei die Zusammensetzung von Amplifikationsprimern eine Vielzahl von Amplifikationsprimerpaaren mit dualen Bibliotheksmarkierungen umfasst, wobei jedes Amplifikationsprimerpaar eine 5'-Ende-Bibliotheksmarkierung und eine 3'-Ende-Bibliotheksmarkierung umfasst,
wobei die Längen von mehrfachen 5'-Ende-Bibliotheksmarkierungen der Amplifikationsprimerpaare alle gleich sind, und die Längen von mehrfachen 3'-Ende Bibliotheksmarkierungen der Amplifikationsprimerpaare alle gleich sind, und die Vorkommen von jeder Base an derselben Position auch alle gleich sind,
wobei es in der Zusammensetzung wenigstens 3 Basenunterschiede zwischen jeweils zwei beliebigen Bibliotheksmarkierungen gibt, und die Anzahl von aufeinanderfolgenden gleichen Basen in jeder Bibliotheksmarkierung nicht größer als 3 ist, und der GC-Gehalt in allen Bibliotheksmarkierungen jeweils 40-60 % beträgt,
wobei die Zusammensetzung eine Kombination von 4n 4-balancierten Amplifikationsprimerpaaren umfasst, oder eine Kombination aus 8n 8-balancierten Amplifikationsprimerpaaren, wobei n eine ganze Zahl größer als oder gleich 1 ist,
wobei in der Kombination von 4n 4-balancierten Amplifikationsprimerpaaren die 5'-Ende-Bibliotheksmarkierungen ausgewählt sind aus einer beliebigen oder mehreren der 96 in Tabelle 1 gezeigten Gruppen, und die 3'-Ende-Bibliotheksmarkierungen ausgewählt sind aus einer beliebigen oder mehreren der 96 in Tabelle 1 gezeigten Gruppen, die verschieden von den 5'-Ende-Bibliotheksmarkierungen sind;
wobei in der Kombination von 8n 8-balancierten Amplifikationsprimerpaaren die 5'-Ende-Bibliotheksmarkierungen ausgewählt sind aus einer beliebigen oder mehreren der 48 in Tabelle 2 gezeigten Gruppen, und die 3'-Ende-Bibliotheksmarkierungen ausgewählt sind aus einer beliebigen oder mehreren der 48 in Tabelle 2 gezeigten Gruppen, die verschieden von den 5'-Ende-Bibliotheksmarkierungen sind;
wobei jedes Amplifikationsprimerpaar ferner eine universelle Amplifikationssequenz am 5'-Ende und eine universelle Amplifikationssequenz am 3'-Ende umfasst, wobei die universelle Amplifikationssequenz am 5'-Ende eine universelle Upstream-Sequenz der 5'-Ende-Bibliotheksmarkierung und eine universelle Downstream-Sequenz der 5'-Ende-Bibliotheksmarkierung umfasst, und die universelle Amplifikationssequenz am 3'-Ende eine universelle Upstream-Sequenz der 3'-Ende-Bibliotheksmarkierung und eine universelle Downstream-Sequenz der 3'-Ende-Bibliotheksmarkierung umfasst;
wobei die universelle Upstream-Sequenz der 5'-Ende-Bibliotheksmarkierung SEQ ID NO: 793 ist, und die universelle Downstream-Sequenz der 5'-Ende-Bibliotheksmarkierung SEQ ID NO: 794 ist; die universelle Upstream-Sequenz der 3'-Ende Bibliotheksmarkierung SEQ ID NO: 795 ist, und die universelle Downstream-Sequenz der 3'-Ende Bibliotheksmarkierung SEQ ID NO: 796 ist; oder
die universelle Upstream-Sequenz der 5'-Ende-Bibliotheksmarkierung SEQ ID NO: 793 ist, und die universelle Downstream-Sequenz der 5'-Ende-Bibliotheksmarkierung SEQ ID NO: 797 ist; die universelle Upstream-Sequenz der 3'-Ende Bibliotheksmarkierung SEQ ID NO: 795 ist, und die universelle Downstream-Sequenz der 3'-Ende-Bibliotheksmarkierung SEQ ID NO: 798 ist;
wobei das Kit ferner Bubble-Adapter umfasst, wobei die Bubble-Adapter eine erste Adaptersequenz und eine zweite Adaptersequenz umfassen, die erste Adaptersequenz SEQ ID NO: 769 ist, und die zweite Adaptersequenz SEQ ID NO: 770 ist, die zusammen mit dem Amplifikationsprimerpaar verwendet werden, das die SEQ ID NO: 793, SEQ ID NO: 794, SEQ ID NO: 795 und SEQ ID NO: 796 umfasst;
oder
die erste Adaptersequenz SEQ ID NO: 773 ist, und die zweite Adaptersequenz 774 ist, die zusammen mit dem Amplifikationsprimerpaar verwendet werden, das die SEQ ID NO: 793, SEQ ID NO: 797, SEQ ID NO: 795 und SEQ ID NO: 798 umfasst.

2. Verfahren zum Konstruieren einer Sequenzierungsbibliothek basierend auf einer MGI-Sequenzierungsplattform, umfassend Anwenden des Kits nach Anspruch 1 zum Konstruieren einer Bibliothek.

3. Sequenzierungsbibliothek, umfassend eine beliebige der Kombinationen von Amplifikationsprimern und der Blasenadapter in dem Kit nach Anspruch 1.

## Revendications

1. Kit de construction de bibliothèque de séquençage, comprenant une composition d'amorces d'amplification comportant des étiquettes de bibliothèque doubles sur la base d'une plate-forme de séquençage MGI,
la composition d'amorces d'amplification comprenant une pluralité de paires d'amorces d'amplification comportant des étiquettes de bibliothèque doubles, chaque paire d'amorces d'amplification comprenant une étiquette de bibliothèque d'extrémité 5' et une étiquette de bibliothèque d'extrémité 3',
les longueurs de plusieurs étiquettes de bibliothèque d'extrémité 5' des paires d'amorces d'amplification étant toutes les mêmes, et les longueurs de plusieurs étiquettes de bibliothèque d'extrémité 3' des paires d'amorces d'amplification étant toutes les mêmes, et les occurrences de chaque base au niveau de la même position étant également toutes les mêmes,
dans la composition, au moins 3 différences de base existant entre deux étiquettes de bibliothèque quelconques, et le nombre de bases identiques continues dans une quelconque étiquette de bibliothèque ne dépassant pas 3, des teneur en GC dans toutes les étiquettes de bibliothèque étant toutes de 40 à 60 %,
la composition comprenant une combinaison de paires d'amorces d'amplification équilibrées en 4 4n, ou une combinaison de paires d'amorces d'amplification équilibrées en 8 8n, n étant un entier supérieur ou égal à 1,
dans la combinaison de paires d'amorces d'amplification équilibrées en 4 4n, les étiquettes de bibliothèque d'extrémité 5' étant choisies parmi l'un quelconque ou plusieurs des 96 groupes présentés dans le tableau 1, et les étiquettes de bibliothèque d'extrémité 3' étant choisies parmi l'un quelconque ou plusieurs des 96 groupes présentés dans le tableau 1 qui sont différents des étiquettes de bibliothèque d'extrémité 5';
dans la combinaison de paires d'amorces d'amplification équilibrées en 8 8n, les étiquettes de bibliothèque d'extrémité 5' étant choisies parmi l'un quelconque ou plusieurs quelconques des 48 groupes présentés dans le tableau 2, et les étiquettes de bibliothèque d'extrémité 3' étant choisies parmi l'un quelconque ou plusieurs quelconques des 48 groupes présentés dans le tableau 2 qui sont différentes des étiquettes de bibliothèque d'extrémité 5';
chaque paire d'amorces d'amplification comprenant en outre une séquence d'amplification universelle d'extrémité 5' et une séquence d'amplification universelle d'extrémité 3', la séquence d'amplification universelle d'extrémité 5' comprenant une séquence amont universelle de l'étiquette de bibliothèque d'extrémité 5' et une séquence aval universelle de l'étiquette de bibliothèque d'extrémité 5', et la séquence d'amplification universelle d'extrémité 3' comprenant une séquence amont universelle de l'étiquette de bibliothèque d'extrémité 3' et une séquence aval universelle de l'étiquette de bibliothèque d'extrémité 3' ;
la séquence amont universelle de l'étiquette de bibliothèque d'extrémité 5' étant la SEQ ID NO: 793, et la séquence aval universelle des étiquettes de bibliothèque d'extrémité 5' étant la SEQ ID NO: 794 ; la séquence amont universelle de l'étiquette de bibliothèque d'extrémité 3' étant la SEQ ID NO: 795, et la séquence aval universelle de l'étiquette de bibliothèque d'extrémité 3' étant la SEQ ID NO: 796 ; ou
la séquence amont universelle de l'étiquette de bibliothèque d'extrémité 5' étant la SEQ ID NO: 793, et la séquence aval universelle de l'étiquette de bibliothèque d'extrémité 5' étant la SEQ ID NO: 797 ; la séquence amont universelle de l'étiquette de bibliothèque d'extrémité 3' étant la SEQ ID NO: 795, et la séquence aval universelle de l'étiquette de bibliothèque d'extrémité 3' étant la SEQ ID NO: 798 ;
le kit comprenant en outre des adaptateurs à bulle, les adaptateurs à bulle comprenant une première séquence d'adaptateur et une deuxième séquence d'adaptateur, la première séquence d'adaptateur étant la SEQ ID NO: 769, et la deuxième séquence d'adaptateur étant la SEQ ID NO: 770, qui sont utilisées ensemble avec la perte d'amorces d'amplification comprenant les SEQ ID NO: 793, SEQ ID NO: 794, SEQ ID NO: 795 et SEQ ID NO: 796 ;
ou
la première séquence d'adaptateur étant la SEQ ID NO: 773, et la deuxième séquence d'adaptateur étant la SEQ ID NO: 774, qui sont utilisées ensemble avec la paire d'amorces d'amplification comprenant les SEQ ID NO: 793, SEQ ID NO: 797, SEQ ID NO: 795 et SEQ ID NO: 798.

2. Procédé pour la construction d'une bibliothèque de séquençage sur la base d'une plate-forme de séquençage MGI, comprenant une application du kit selon la revendication 1 pour construire une bibliothèque.

3. Bibliothèque de séquençage, comprenant l'un quelconque parmi la combinaison d'amorces d'amplification et les adaptateurs à bulle dans le kit selon la revendication 1.
